# EUROPEAN PATENT SPECIFICATION

(11) **EP 4 161 402 B1**
(45) Date of publication and mention of the grant of the patent: **06.08.2025**
(21) Application number: 21822376.6
(22) Date of filing: 07.06.2021
(51) Int. Cl.: A61B 17/04, A61B 17/00, A61B 17/03, A61B 17/06, A61B 17/062, A61B 17/28

(54) **JOINTED DEVICE FOR AUTOMATIC SUTURE**
ZUSAMMENGEFÜGTE VORRICHTUNG FÜR AUTOMATISCHE NAHT
DISPOSITIF ARTICULÉ POUR SUTURE AUTOMATIQUE

(30) Priority: 07.06.2020 US 202063035820 P
(43) Date of publication of application: 12.04.2023
(73) Proprietor: Health Research, Inc., Buffalo, NY 14263 (US)
(72) Inventor: GURU, Khurshid, Amherst, NY 14051 (US)
(74) Representative: Kramer, Dani
(86) International application number: PCT/US2021/036271
(87) International publication number: WO 2021/252404

(56) References cited:
- WO-A1-2020/092656
- US-A1- 2009 240 264
- US-A1- 2010 114 122
- US-A1- 2013 296 955
- US-A1- 2013 296 955
- US-A1- 2018 280 019
- US-B1- 6 626 917

## Description

### Cross-Reference to Related Applications

This application claims priority to U.S. Provisional Application No. 63/035,820, filed on June 7, 2020.

### Field of the Disclosure

The present disclosure relates to suturing, and in particular for suturing during a minimally-invasive procedure.

### Background to the Disclosure

US2013296955A1 relates to a device for use in arthroscopic repair surgery and more specifically for use in repairing rotator cuff tendon tears in the shoulder. The device is used to place one or more sutures within the tendon in a grasping-type stitch. The suture may then be secured to the humerus bone via a bone anchor or tunnel allowing the tendon to heal.

US2010114122A1 relates to a sewing device for overcasting a mechanical twisted suture. The device comprises a body provided with a spiral needle which is rotatable and translationally displaceable and is connected to a drive for rotating it, clamping jaws which are arranged on the distal end of the body for fixing the tissue parts to be sewed and provided with a unit for controlling the relative position thereof. The spiral needle is designed in the form of an atraumatic or a hollow needle, is provided with a thread arranged therein, comprises at least two complete turns and is positioned in such a way that it is displaceable in a direction along the clamping jaws at a distance equal to or greater than the entire length of the spiral. The device comprises a nest for catching the pointed end of the needle and/or the thread at the end of the suture and means for extracting the needle from the tissue.

### Brief Summary of the Disclosure

The invention is defined by the appended set of claims. According to the invention, there is provided a device for automatic suturing. The device includes a shaft having a longitudinal axis. The shaft has a joint rotatable about a joint axis which is at an angle > 0° to the longitudinal axis. The device includes a clamp at a distal end of the shaft. The clamp is configured to be positioned over a length of tissue to be sutured. A driver is configured to engage with a screw of the shaft so that the driver translates along a length of the shaft when the driver is rotated. A helical needle is connected to the driver such that the helical needle is rotated while advancing or retracting over a length of the clamp and over the joint of the shaft. In this way, rotation of the driver causes rotation of the helical needle. In some embodiments, the helical needle further comprises one or more windows. In some embodiments, a tip of the helical needle is at a radially exterior portion of the helical needle for movement over the joint of the shaft. The helical needle may be configured to be advanced over an exterior of the clamp.

In some embodiments, the device further comprises a suturing thread having a leading end near a tip of the helical needle. The helical needle may be hollow and the suturing thread is disposed through the helical needle. The device may further comprise at least one cutter for severing the suturing thread. The suturing thread has a diameter, the helical needle has an inside diameter, and the ratio of the suturing thread diameter to the helical needle inside diameter is in the range of 1:6-1:10, inclusive. In some embodiments, the ratio of the suturing thread diameter to the helical needle inside diameter is greater than 1:8.

The device may further have a lock for reversibly locking the clamp in a closed position. The device may further have a shaft attached to the clamp, and the helical needle may be configured to be disposed over the shaft when in a retracted position. In some embodiments, a motor is operably connected to the driver.

In another aspect of the present disclosure, there is provided a method for automatic suturing. The method includes engaging a clamp of a device according to any one of the disclosed embodiments over a first portion of tissue to be sutured. The clamp may be locked in place (e.g., locked in a closed position). The helical needle of the device is advanced along a length of the clamp such that the helical needle passes through the tissue to be sutured. A leading end of a suturing thread is attached near a tip of the helical needle. In this way, the suturing thread is pulled through the tissue with the helical needle. The method includes capturing the leading end of the suturing thread and retracting the helical needle back through the tissue so as to leave the suturing thread in place. The clamp may then be disengaged from the tissue.

The leading end of the suturing thread and/or a trailing end of the suturing thread may be fixed to form one or more sutures. For example, the leading end of the suturing thread and/or the trailing end of the suturing thread may be fixed by knotting.

The needle may be advanced using, for example, a motor (e.g., an electric motor). The helical needle may be advanced continuously, or selectively (e.g., in single rotation increments).

In another aspect of the present disclosure, there is provided a method for automatic suturing. The method includes engaging a clamp over a length of tissue to be sutured. In some embodiments, the clamp may be locked in place over the tissue (e.g., over the length of tissue). A helical needle is advanced along a length of the clamp such that the helical needle passes through the tissue to be sutured. A leading end of a suturing thread may be attached at a tip of the helical needle. The leading end of the suturing thread is captured, for example, when the tip of the helical needle is advanced to an end position. In some embodiments, a removable tip of the needle is captured with the leading end of the suturing thread. In such embodiments, the helical needle is retracted without the tip. The helical needle is retracted back through the tissue so as to leave the thread in place. The method includes disengaging the clamp from the tissue.

The leading end of the thread may be fixed to form one or more sutures. Similarly, a trailing end of the suturing thread may be fixed to form one or more suture. In some embodiments, both the leading end and the trailing end are fixed to form one or more suture. For example, sutures may be formed at each of the leading end and the trailing end. The leading end of the suturing thread and/or the trailing end of the suturing thread may be fixed using knots (e.g., by knotting the ends).

The method may include cutting the leading end of the suturing thread so as to separate the tip of the helical needle from a thread portion to remain in place following retraction of the helical needle (*e.g.,* a thread portion forming a suture).

The helical needle may be advanced and/or retracted by an electric motor. The helical needle may be advanced in single rotation increments. The helical needle may be advanced in increments less than a full rotation. The helical needle may be advanced in increments greater than a full rotation. The helical needle may be advanced to the end position.

In another aspect, the present disclosure may be embodied as a device for automatic suturing. The device comprises a clamp. The clamp is configured to be positioned over a length of tissues to be sutured. A lock may be provided for reversibly locking the clamp in a closed position. A helical needle is configured to be advanced and retracted along a length of the clamp. For example, the helical needle may be advanced along an interior portion of the clamp. In another example, the helical needle may be advanced over an exterior portion of the clamp. The needle has a tip, and a suturing thread having a leading end is attached to the tip of the helical needle. The needle may be hollow and the thread may be disposed through the needle.

The device may include a trap configured to capture a leading end of the thread once the helical needle has been advanced along at least a portion of the length of the clamp. The tip of the needle may be removable. The trap may be configured to capture the tip of the needle with the leading end of the thread.

The clamp may include a helical guide configured to guide the helical needle during advancement and retraction. The guide may be configured on an interior portion of the clamp. The device may further include at least one cutter for severing the suturing thread.

### Description of the Drawings

For a fuller understanding of the nature and objects of the disclosure, reference should be made to the following detailed description taken in conjunction with the accompanying drawings, in which:
Figure 1 is a diagram depicting a device according to an embodiment of the present disclosure;

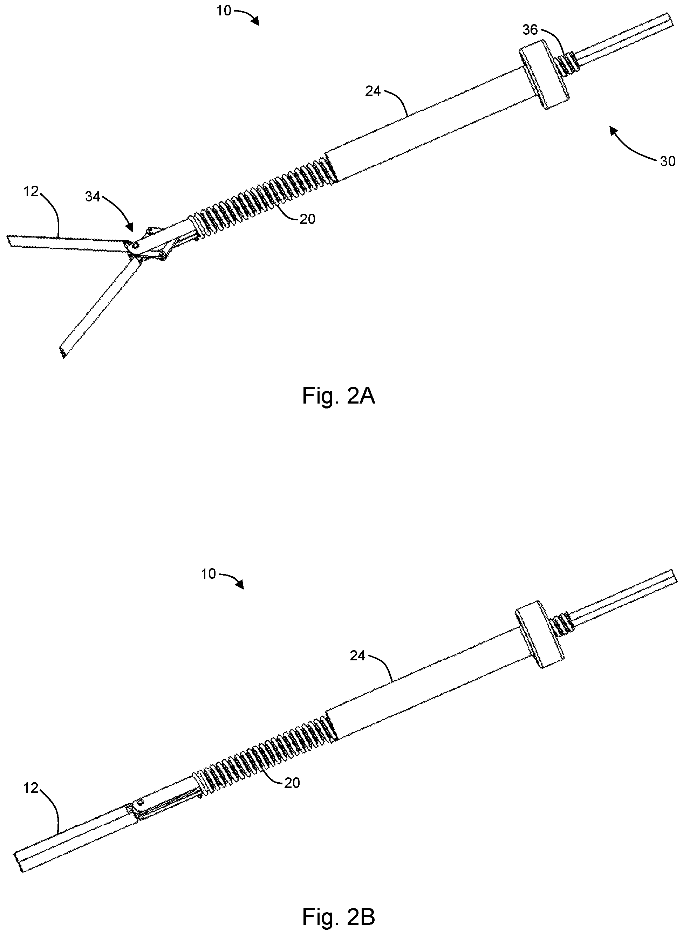

Figure 2A is a diagram of the device of Figure 1, wherein the clamp is shown in an open configuration;
Figure 2B is a diagram of the device of Figures 1-2A, wherein the clamp is shown closed;
Figure 3A is a diagram showing a detail view of a clamp of the device of Figures 1-2B, wherein the clamp is shown in an open configuration;
Figure 3B is a diagram showing a detail view of the clamp of the device of Figures 1-3A, wherein the clamp is shown closed;
Figure 4 is a diagram of the device of Figures 1-3B, wherein a needle has been advanced over the clamp;
Figure 5 is a diagram showing a detail of the clamp and needle of Figure 4;
Figure 6 shows a needle according to another embodiment of the present disclosure;
Figure 7 shows a detail view of a tip of the needle of Figure 6;

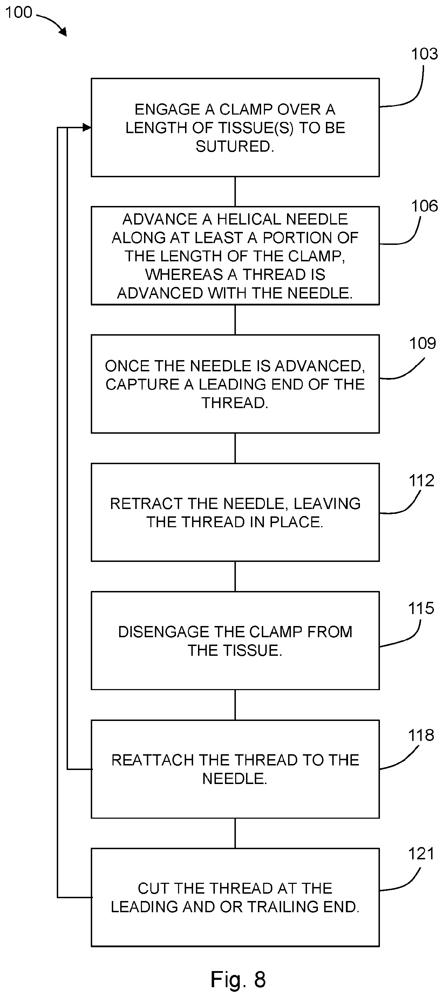

Figure 8 is a chart showing a method of the present disclosure;
Figure 9 is a diagram of device according to another embodiment of the present disclosure;
Figure 10A is a detail view of the driver and needle of the device of Figure 9;
Figure 10B is a partial exploded view of the driver and needle of Figure 10A; and
Figure 11 is a diagram depicting a driver head and needle according to another embodiment of the present disclosure.

### Detailed Description of the Disclosure

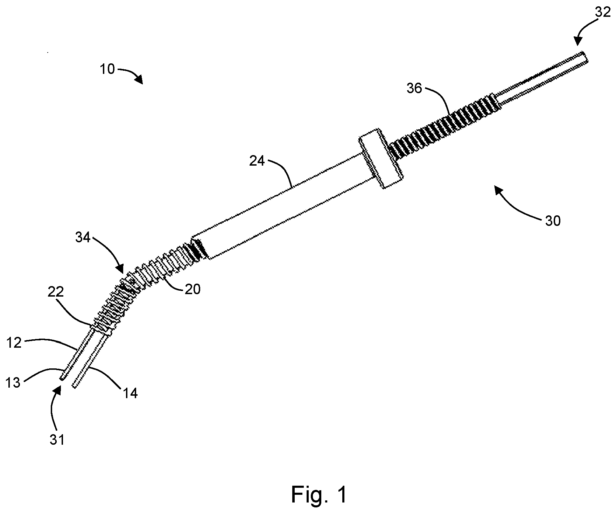

With reference to Figure 1, in an aspect, the present disclosure may be embodied as a device **10** for automatic suturing. The device **10** includes a shaft **30** having a longitudinal axis. A clamp **12** is located at a distal end **31** of the shaft. In an exemplary embodiment, the clamp **12** includes a first side portion **13** and a second side portion **14,** each hingedly connected to the other. A spring may cause the first and second side portions **13,14** to be biased toward each other-*i.e*., biased to a "closed" state of the clamp **12.** Such a clamp **12** is configured to be opened and positioned over a length of tissue. The clamp **12** may be engaged by allowing the clamp to return to the closed position (*e.g*., partially closed as the tissue(s) may now cause some separation at an opening of the clamp **12).** In other embodiments, the clamp is not spring biased and the clamp is operably moved from an open state to the closed state and varying degrees therebetween by a user.

In some embodiments, the device **10** includes a joint **34** at a location of the shaft **30** proximally-located with respect to the clamp **12.** In this way, a distal portion of the shaft including at least the clamp is able to rotate into a position off of *(i.e.,* at an angle > 0° to) the longitudinal axis of the shaft. For example, the joint may have a rotational axis which is orthogonal to the longitudinal axis of the shaft. A joint control member is disposed through the shaft such that the joint is operable from a proximal end **32** of the shaft.

The device **10** further comprises a helical needle **20** movably disposed over a portion of the shaft **30** and/or the clamp **12.** A driver **24** is connected to a proximal end **22** of the needle **20** such that rotation of the driver **24** about the longitudinal axis of the shaft will cause the needle **20** to rotate about the shaft. Similarly, translation of the driver **24** along the length of the shaft will cause the needle **20** to translate as well. The shaft **30** includes a screw **36** with which the driver **24** is engaged. In this way, rotation of the driver will cause the driver to advance along the screw (or withdrawn if rotated in an opposite manner) thereby also translating the driver (and needle) along the length of the shaft. The screw may have a trapezoidal thread form, (for example, an ACME thread profile) or another thread form. The driver may have a length such that it can be manually operated from outside of the body when in use. In some embodiments, the driver may be advanced using a motor, such as an electric motor. In such embodiments, the driver may have a shorter length such that the driver is completely contained within the body when in use, or a longer length such that at least a portion of the driver is located external to the body when in use.

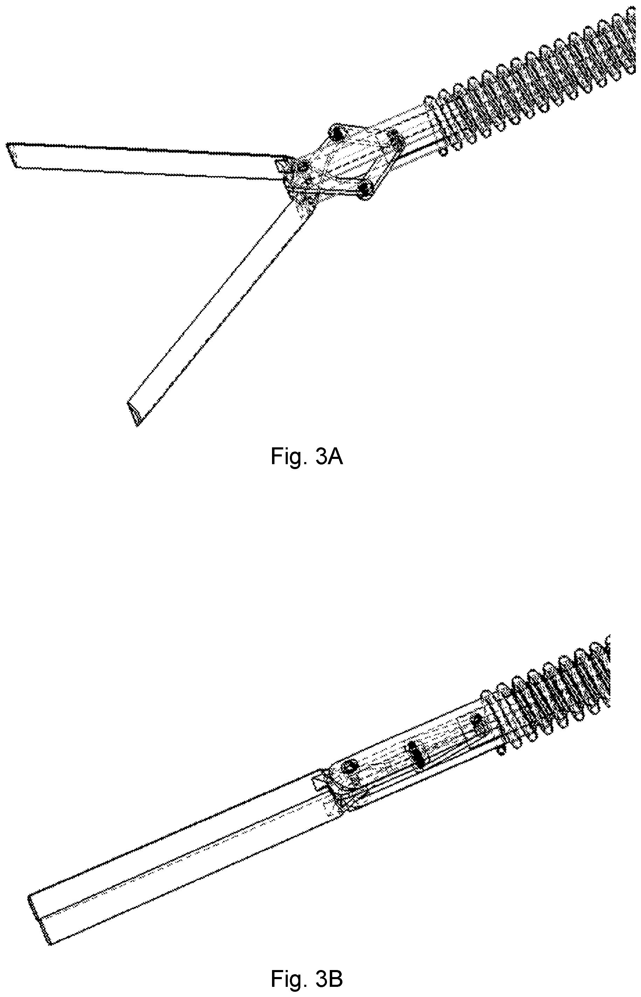

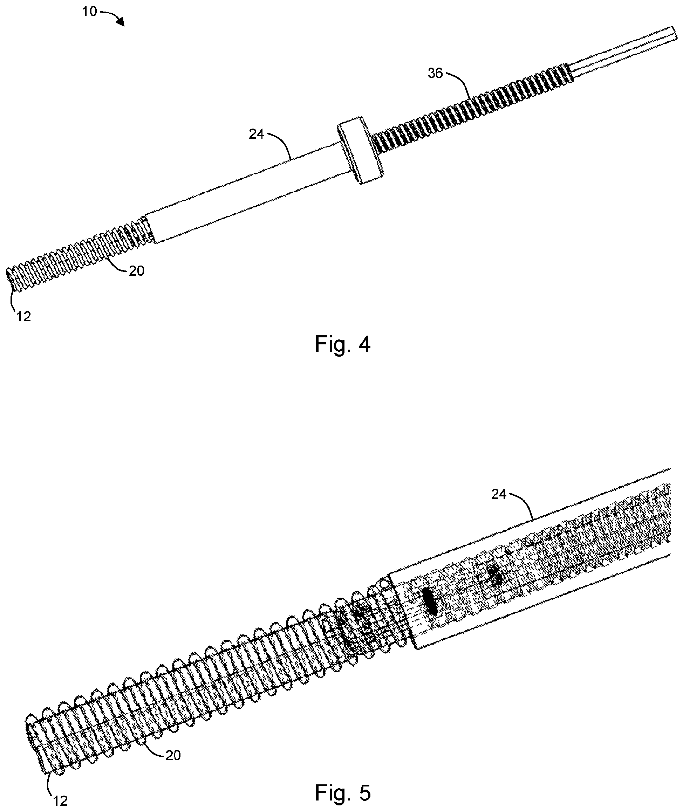

The helical needle **20** is configured such that advancement causes the needle to pierce material (e.g., one or more tissue(s)) contained within the clamp. The needle will pierce the material one or more times as it rotates along the length of the clamp. In some embodiments, the helical needle **20** may be disposed over the shaft **30** when in a withdrawn (retracted) position (Figures 3A and 3B). In this way, the clamp **12** may be opened (Figure 3A) and closed (Figure 3B) over the tissue(s). When the clamp **12** is closed (clamped) on the tissue(s) to be sutured, the helical needle **20** may be advanced along a length of the clamp **12** *(see, e.g.,* Figures 4 and 5)-*i.e.,* along the longitudinal length of the clamp **12.** For example, the helical needle may be advanced over an exterior of the clamp. The shaft and/or the clamp may include a guide or guides (not shown) to guide the helical needle during advancement and retraction. The device may further include a sheath configured to enclose at least a portion of the helical needle when in a retracted position.

The needle **20** is configured such that the needle may be advanced over the clamp **12** when the joint **34** is straight *(i.e.,* such that the clamp is coaxial with the longitudinal axis of the shaft **30)** or when the j oint **34** is bent *(i.e.,* at an angle > 0° from the longitudinal axis of the shaft **30).** For example, the needle may be made from metal (*e.g.*, surgical-grade steel), or rigid plastics or composites (*e.g.*, carbon fiber). A diameter of the needle may also be configured for advancement over the bent j oint. In a non-limiting, experimental embodiment, it has been found that a surgical steel needle with a diameter of 0.065" was able to negotiate advancement over a bent joint of the shaft. In some embodiments, a tip **22** of the needle may be cut *(i.e.,* sharpened) at an angle such that a point of the tip is at a radial exterior of the helix form (see Figure 4). In experimental embodiments, the radial exterior needle tip has proven advantageous when used with a bent joint of the shaft.

The needle **20** may have a gauge of, for example, between 20 G and 10 G (Birmingham gauge; approximately 0.91-3.4 mm, outer diameter), inclusive, but may be smaller or larger as desired for the application. The overall diameter of the helix shape may be, for example, between 0.25-2.0 in (approximately 6.35-50.8 mm), inclusive, but may be smaller or larger as desired for a particular application. In a particular embodiment, a device is designed for use with a 15 mm port size. As such, the helix shape has a diameter which is less than 15 mm. Generally, an overall diameter of the device (diameter of the shaft, clamp, needle, driver, etc.) is less than a port size used in a particular procedure.

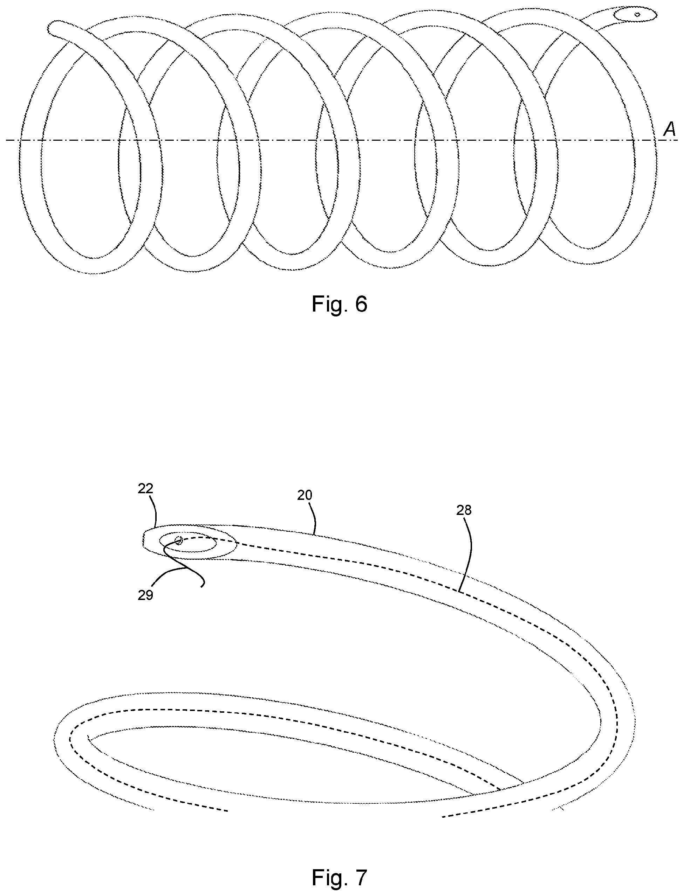

With reference to Figure 7, the device **10** further includes a suturing thread **28** for suturing the tissue contained in the clamp **12.** The suturing thread **28** has a leading end **29** which may be attached to the needle **20** *(see, e.g.,* Figure 4). In some embodiments, the leading end **29** is attached to *(i.e.,* near) the tip **22** of the needle **20.** In an example, the suturing thread **28** may be attached by way of a spring-loaded, multi-prong grasper as is known in the art. In other embodiments, the suturing thread **28** is attached by way of being tied to an eye of the needle **20.** In other embodiments, the suturing thread is attached to the tip of the needle by nature of being disposed through a lumen of the needle. Other techniques for attachment including, for example, adhesives, will be apparent in light of the present disclosure. In some embodiments, the needle **20** is hollow and the suturing thread **28** is disposed through the hollow portion (lumen) of the needle **20** emerging at the tip **22.** In other embodiments, the suturing thread may be generally external to the needle and attached at the tip. The needle **20** and suturing thread **28** are configured such that as the needle **20** is advanced along a length of the clamp-for example, through the guide **18** of the clamp **12-and** through portions of the tissue(s) held by the clamp **12,** the suturing thread **28** is carried through the tissue as well.

The number of sutures possible through the held tissue will generally match the number of helixes. For example, if a helix-shaped needle **20** includes 24 helixes, there will generally be a result of up to 24 sutures (assuming tissue was properly placed in the clamp **12,** etc.) Note that the resulting suture is not 24 individual sutures, but a running suture or "continuous *stitch"-i.e.,* a continuous suture having only the end stitches tied off. The needle **20** may have, for example, between 12 and 24 helixes, inclusive, but may have more helixes or fewer helixes. The needle **20** may have a length of, for example, between 3 inches and 6 inches (approximately 76.2-152.4 mm), inclusive, but may be longer or shorter.

The device **10** may include a trap **40** configured to engage with the tip **22** of the needle **20** when the needle **20** has been completely advanced through the clamp **12.** By engage the needle **20,** it is intended that the trap **40** is configured to capture the suturing thread **28***-e.g.,* at least a portion of the thread, for example, at or near the leading end **29** of the suturing thread **28.** In some embodiments, the trap **40** is configured such that the tip **22** of the needle **20** enters the trap **40.** In some embodiments, the tip **22** is removable, and the trap **40** may be configured to remove and retain the tip **29** (along with at least a portion of the leading end **29** of the thread) within the trap **40.** By capturing the suturing thread **28,** the helical needle **20** may be retracted through the clamp **12** while leaving the suturing thread **28** in place. In other embodiments, the suturing thread may be held using another tool (*e.g.*, laproscopic tool, etc.) while the needle is retracted.

In some embodiments, the once retracted, leaving the suture thread **28** in place, the thread **28** may be reattached to needle, such as, for example, at the tip **22** of the needle **20** *(i.e.,* at a portion of the thread spaced apart from the in-place portion of the thread). The needle may include a feature, such as, for example, a hook, a slit, a clamp, or other etc. to which the thread may be attached. For example, the thread may be reattached by tying the thread to the needle (*e.g.,* to a feature of the needle). Once the thread is reattached to the needle, the needle can be re-advanced through the tissue. For example, another portion of the tissue(s) can be clamped and the suture can continue from the earlier, in-place suture. In some embodiments, the suturing thread is disposed through the shaft and into an end of the needle engaged with the driver. In some embodiments, the suturing thread enters the needle at a location of the needle near the driver.

In some embodiments, the device **10** may further include one or more cutters to sever the suturing thread at the leading end and/or a trailing end. In this way, the device **10** may be removed from the body while leaving the suturing thread in place through the tissue(s).

In another aspect of the present disclosure there is provided a method **100** for automatic suturing. For example, the method **100** may be used to create a running suture over a length of tissue (or tissues). A clamp is engaged **103** over a length of tissue (or tissues) to be sutured. For example, where bowel may need to be sutured closed over a length of the bowel, the clamp is positioned over the length of the bowel such that the two tissue portions to be sutured together are within the clamp. The clamp may be engaged **103** to retain the tissue therein. The clamp may be engaged **103** using a lock configured to prevent the clamp from opening.

A helical needle is advanced **106** along at least a portion of a length of the clamp. For example, the needle may be advanced over the clamp with or without a guide or guides. The clamp and the helical needle may be of any configuration as defined herein. The helical needle is advanced **106** in a corkscrew-like fashion*-i.e.,* advanced by rotating the needle about a central longitudinal axis of the helix shape. By advancing along at least a portion of the length of the clamp, the needle passes through the tissue(s) to be sutured. Based on the clamp and needle configuration and/or the manner of clamping the tissue(s) in the clamp, the needle may pass through the tissue(s) once with each rotation of the needle or twice with each rotation of the needle. The needle may be advanced by an actuator which is controlled by a surgeon. For example, each time the actuator is triggered (*e.g.*, squeezing a trigger, depressing a button, toggling a switch, depressing a foot pedal, etc.), the needle may advance by one rotation and may puncture the tissue(s) one or two times.

The needle is a hollow needle and includes a thread (a suturing thread) disposed at least partially through the hollow interior of the needle. In this way, the suturing thread is carried through the clamp and tissue by the helical needle. The suturing thread is outside of the needle and attached to a tip portion of the needle. In this way, the suturing thread is pulled through the tissue by needle as the needle is advanced **106** through the clamp. The needle is advanced **106** one rotation at a time *(i.e.,* with each actuation of the device). Actuating the device may advance the needle by less than one rotation. For example, the actuator advances the needle by half of a rotation, a third of a rotation, a quarter of a rotation, or any other rotation less than a full rotation. As such, the tissue may be punctured by the needle one or two times for each actuation of the device or not punctured at all by actuation. The needle is advanced **106** by more than one rotation at a time. For example, a surgeon may cause the needle to advance, and such advancement may continue until the needle has travelled through its entire path through the clamp. The advancement **106** of the needle may occur only while activated by an operator. For example, a surgeon may squeeze a trigger to cause the needle to advance and the needle may continue to advance until the surgeon releases the trigger. The needle may advance at a selectively variable rate. For example, the advancement rate may be proportional to a position of an actuator (*e.g.*, variable trigger, foot pedal position, etc.) In another example, the advancement rate is determined according to a control that is separate from the actuator such as, for example, a multi-setting switch, dial, rotatable knob, etc. Other advancement schemes will be apparent in light of the present disclosure.

A leading end of the suturing thread may be captured **109** or otherwise fixed at a location of the tissue once the needle is advanced **106** along the clamp. For example, the suturing thread may be captured when the tip of the helical needle is advanced to an end position. The end location may be at a distal end of the clamp. The end location may be at a position spaced apart from the distal end of the clamp. The end location may be selected by a user according to the application. For example, as further described below, a trap is positioned on the clamp to capture **109** at least a portion of the suturing thread. The tip of the needle is removable, and the tip is captured **109** along with the at least a portion of the suturing thread. Alternatively, the suturing thread may be captured manually by an operator (for example, a surgeon) using a separate device.

Once the suturing thread is captured **109,** the needle is retracted **112** back along the length clamp. For example, the needle is retracted **112** using an opposite corkscrew movement from the advancing movement-i.e., by oppositely-rotating the needle about the central longitudinal axis of the helix shape. Because at least a portion of the suturing thread is captured **109,** the suturing thread is left in place during retraction **112** of the needle. By left in place, it is intended that the suturing thread remain disposed through the tissue(s) at the locations where the needle passed through the tissue(s). Where the suturing thread is disposed through a hollow portion of the needle, the needle is retracted **112** by passing over the suturing thread (the suturing thread slides through the hollow portion, thereby remaining in place once the needle is retracted).

The clamp is disengaged **115** from the tissue. The thread may be reattached **118** to the needle, for example, to a tip of the needle. In this way, the device may be immediately used again to perform another suture on the same or a different tissue. By immediately, it is intended that a subsequent suture may be performed without the need to remove the device from the body of the individual. The suturing thread may be cut **121** at the leading end and/or a trailing end such that the clamp and needle may be moved or removed while leaving the suturing thread in place.

The leading end of the suturing thread and/or the trailing end of the suturing thread may be fixed to complete the suture. One or both of the ends are fixed **121** by knotting the thread (securing the thread to itself). The suturing thread is knotted such that one or both ends cannot pull through the tissue. The suturing thread is configured to swell to a thicker diameter (in this case, thicker means a diameter greater than an initial diameter of the suturing thread) thereby retaining the suturing thread in the tissue. Such threads are known in the art. Before fixation of one or both ends, the suturing thread may be adjusted such that the suture is tightened as desired. One or both ends of the suturing thread may be fixed before or after disengagement **115** of the clamp.

The helical needle is advanced **106** and/or retracted **112** using an electric motor. The helical needle is advanced **106** and/or retracted **112** using by way of a manual operation (*e.g.*, handle, hand crank, etc.)

It has been found that, in some cases, a thread diameter may advantageously be sufficiently smaller than an inside diameter of the needle in order be more easily moved through the needle. For example, when using a 16RW hypodermic tube (having an inside diameter of 0.047 inches (1.19 mm) and an outside diameter of 0.065 inches (1.65 mm)) as the needle, thread sizes of 4-0 (0.15 mm diameter) or smaller provide satisfactory performance. A 4-0 poly suture was successfully used with a helical needle formed from 16RW stainless steel hypodermic tubing. Other ratios of suture diameter to needle insider diameter may be acceptable and may depend on one or more of the suture material, the needle material, the needle helix parameters (helix diameter, helix angle), etc. Acceptable ratios of suture diameter:needle inside diameter may range from 1:6 to 1:10, inclusive, and all values therebetween. The ratio may be even higher or lower than these exemplary values/ranges. The ratio of the suturing thread diameter to the helical needle inside diameter is greater than 1:8 (e.g., 1.81, 1.82, ..., 1:9, 1.91, ..., 1:10, etc.)

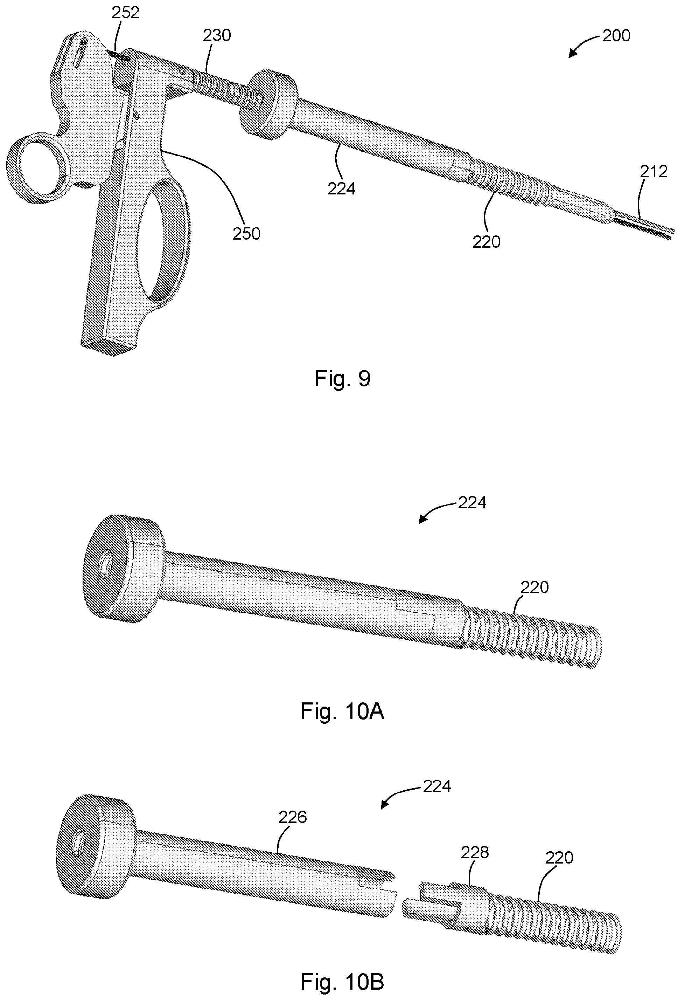

Figure 9 depicts another embodiment of a device **200** for automatic suturing. The device **200** includes a shaft **230** with a screw thread for advancing a driver **224** when the driver **224** is rotated. The driver **224** is configured to drive a needle **220** as described above. A clamp **212** is operable to secure tissue for suturing when the needle **220** is advanced over the clamp **212.** The device **200** also includes a handle **250** which is configured to operate the clamp **212** using a rod **252.** Figures 10A and 10B show a detail view of the driver **224** and needle **220.** The embodiment of device **200** utilizes a two-piece driver **224** having a driver body **226** and a driver head **228.** The driver body **226** is configured to rotate the driver head **228,** but the design allows the driver body to advance at a rate that is different than the driver head. In this way, any differences between the helix configuration and the screw configuration (of the shaft) can be tolerated.

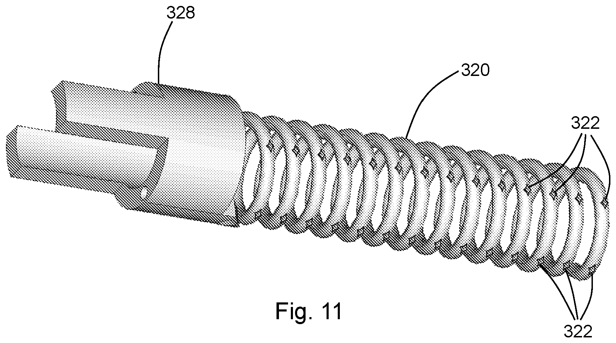

Figure 11 depicts an embodiment of a driver head **328** and needle **320.** The needle of this embodiment includes a plurality of windows **322-**orifices through the needle wall, allowing external access to the lumen of the needle. In this way, a suture thread may be loaded into the needle by sequentially inserting the thread from a window **322** to the next window **322,** and so on until the needle is loaded with suture thread. In the embodiment shown in Figure 11, the needle includes a window **322** every 90° of the helix (i.e., four windows per rotation of the helix). Other embodiments utilize fewer windows or more windows.

Although the present disclosure has been described with respect to one or more particular embodiments, it will be understood that other embodiments of the present disclosure may be made without departing from the scope of the invention as defined in the appended claims.

## Claims

1. A device (10, 200) for automatic suturing, comprising:
a shaft (30, 230) having a longitudinal axis, the shaft (30, 230) having a joint (34) rotatable about a joint axis which is at an angle > 0° to the longitudinal axis;
a clamp (12, 212) at a distal end (31) of the shaft (30, 230) and configured to be positioned over a length of tissue to be sutured;
a driver (24, 224) configured to engage with a screw (36) of the shaft (30, 230) so as to translate along a length of the shaft (30, 230) when rotated;
a helical needle (20, 220) connected to the driver (24, 224) such that the helical needle (20, 220) is rotated while advancing or retracting over a length of the clamp (12, 212) and over the joint (34) of the shaft (30, 230).

2. The device (10, 200) of claim 1, wherein a tip (22) of the helical needle (20, 220) is at a radially exterior portion of the helical needle (20, 220) for movement over the joint (34) of the shaft (30, 230).

3. The device (10, 200) of claim 1, further comprising a suturing thread (28) having a leading end (29) near a tip (22) of the helical needle (20, 220).

4. The device (10, 200) of claim 3, wherein the helical needle (20, 220) is hollow and the suturing thread (28) is disposed through the helical needle (20, 220).

5. The device (10, 200) of claim 3, further comprising at least one cutter for severing the suturing thread (28).

6. The device (10, 200) of claim 3, wherein the suturing thread (28) has a diameter, the helical needle (20, 220) has an inside diameter, and the ratio of the suturing thread (28) diameter to the helical needle (20, 220) inside diameter is in the range of 1:6-1:10, inclusive, preferably wherein the ratio of the suturing thread (28) diameter to the helical needle (20, 220) inside diameter is greater than 1:8.

7. The device (10, 200) of claim 1, further comprising a lock for reversibly locking the clamp (12, 212) in a closed position.

8. The device (10, 200) of claim 1, wherein the helical needle (20, 220) is configured to be advanced over an exterior of the clamp (12, 212).

9. The device (10, 200) of claim 1, wherein the helical needle (20, 220) is configured to be disposed over the shaft (30, 230) when in a retracted position.

10. The device (10, 200) of claim 1, further comprising a motor operably connected to the driver (24, 224).

11. The device (200) of claim 1, wherein the helical needle (220) further comprises one or more windows (322).

## Patentansprüche

1. Vorrichtung (10, 200) zum automatischen Nähen, umfassend:
einen Schaft (30, 230) mit einer Längsachse, wobei der Schaft (30, 230) ein Gelenk (34) aufweist, das um eine Gelenkachse drehbar ist, die in einem Winkel > 0° zu der Längsachse ist;
eine Klemme (12, 212) an einem distalen Ende (31) des Schafts (30, 230) und dazu konfiguriert, über einer zu nähenden Gewebelänge positioniert zu werden;
einen Treiber (24, 224), der dazu konfiguriert ist, eine Schraube (36) des Schafts (30, 230) in Eingriff zu nehmen, um sich bei Drehung entlang einer Länge des Schafts (30, 230) zu verschieben;
eine Spiralnadel (20, 220), die mit dem Treiber (24, 224) verbunden ist, so dass die Spiralnadel (20, 220) gedreht wird, während sie über eine Länge der Klemme (12, 212) und über das Gelenk (34) des Schafts (30, 230) vorgeschoben oder zurückgezogen wird.

2. Vorrichtung (10, 200) nach Anspruch 1, wobei eine Spitze (22) der Spiralnadel (20, 220) an einem radial äußeren Abschnitt der Spiralnadel (20, 220) zur Bewegung über das Gelenk (34) des Schafts (30, 230) ist.

3. Vorrichtung (10, 200) nach Anspruch 1, ferner umfassend einen Nähfaden (28) mit einem vorderen Ende (29) nahe einer Spitze (22) der Spiralnadel (20, 220).

4. Vorrichtung (10, 200) nach Anspruch 3, wobei die Spiralnadel (20, 220) hohl ist und der Nähfaden (28) durch die Spiralnadel (20, 220) angeordnet ist.

5. Vorrichtung (10, 200) nach Anspruch 3, ferner umfassend mindestens eine Schneidvorrichtung zum Durchtrennen des Nähfadens (28).

6. Vorrichtung (10, 200) nach Anspruch 3, wobei der Nähfaden (28) einen Durchmesser aufweist, die Spiralnadel (20, 220) einen Innendurchmesser aufweist und das Verhältnis des Durchmessers des Nähfadens (28) zu dem Innendurchmesser der Spiralnadel (20, 220) im Bereich von 1:6-1:10 einschließlich liegt, vorzugsweise wobei das Verhältnis des Durchmessers des Nähfadens (28) zu dem Innendurchmesser der Spiralnadel (20, 220) größer als 1:8 ist.

7. Vorrichtung (10, 200) nach Anspruch 1, ferner umfassend eine Arretierung zum reversiblen Arretieren der Klemme (12, 212) in einer geschlossenen Position.

8. Vorrichtung (10, 200) nach Anspruch 1, wobei die Spiralnadel (20, 220) dazu konfiguriert ist, über eine Außenseite der Klemme (12, 212) vorgeschoben zu werden.

9. Vorrichtung (10, 200) nach Anspruch 1, wobei die Spiralnadel (20, 220) dazu konfiguriert ist, über dem Schaft (30, 230) angeordnet zu sein, wenn sie in einer zurückgezogenen Position ist.

10. Vorrichtung (10, 200) nach Anspruch 1, ferner umfassend einen Motor, der betriebsfähig mit dem Treiber (24, 224) verbunden ist.

11. Vorrichtung (200) nach Anspruch 1, wobei die Spiralnadel (220) ferner ein oder mehrere Fenster (322) umfasst.

## Revendications

1. Dispositif (10, 200) de suture automatique, comprenant :
un arbre (30, 230) ayant un axe longitudinal, l'arbre (30, 230) ayant une articulation (34) capable de rotation autour d'un axe d'articulation qui est à un angle > 0° par rapport à l'axe longitudinal ;
une pince (12, 212) au niveau d'une extrémité distale (31) de l'arbre (30, 230) et configurée pour être positionnée sur une longueur de tissu à suturer ;
un dispositif d'entraînement (24, 224) configuré pour entrer en prise avec une vis (36) de l'arbre (30, 230) de manière à réaliser une translation le long d'une longueur de l'arbre (30, 230) lorsqu'il est amené à tourné ;
une aiguille hélicoïdale (20, 220) connectée au dispositif d'entraînement (24, 224) de sorte que l'aiguille hélicoïdale (20, 220) est amenée à tourner tout en avançant ou en se rétractant sur une longueur de la pince (12, 212) et sur l'articulation (34) de l'arbre (30, 230).

2. Dispositif (10, 200) selon la revendication 1, dans lequel une pointe (22) de l'aiguille hélicoïdale (20, 220) se trouve au niveau d'une partie extérieure de manière radiale de l'aiguille hélicoïdale (20, 220) pour un mouvement sur l'articulation (34) de l'arbre (30, 230).

3. Dispositif (10, 200) selon la revendication 1, comprenant en outre un fil de suture (28) ayant une extrémité avant (29) près d'une pointe (22) de l'aiguille hélicoïdale (20, 220).

4. Dispositif (10, 200) selon la revendication 3, dans lequel l'aiguille hélicoïdale (20, 220) est creuse et le fil de suture (28) est disposé à travers l'aiguille hélicoïdale (20, 220).

5. Dispositif (10, 200) selon la revendication 3, comprenant en outre au moins un outil de coupe destiné à trancher le fil de suture (28).

6. Dispositif (10, 200) selon la revendication 3, dans lequel le fil de suture (28) a un diamètre, l'aiguille hélicoïdale (20, 220) a un diamètre intérieur, et le rapport du diamètre du fil de suture (28) au diamètre intérieur de l'aiguille hélicoïdale (20, 220) est dans la plage de 1/6 à 1/10, inclus, de préférence dans lequel le rapport du diamètre du fil de suture (28) au diamètre intérieur de l'aiguille hélicoïdale (20, 220) est supérieur à 1/8.

7. Dispositif (10, 200) selon la revendication 1, comprenant en outre un verrou pour verrouiller de manière réversible la pince (12, 212) dans une position fermée.

8. Dispositif (10, 200) selon la revendication 1, dans lequel l'aiguille hélicoïdale (20, 220) est configurée pour être avancée sur un extérieur de la pince (12, 212).

9. Dispositif (10, 200) selon la revendication 1, dans lequel l'aiguille hélicoïdale (20, 220) est configurée pour être disposée sur l'arbre (30, 230) lorsqu'elle est dans une position rétractée.

10. Dispositif (10, 200) selon la revendication 1, comprenant en outre un moteur connecté de manière fonctionnelle au dispositif d'entraînement (24, 224).

11. Dispositif (200) selon la revendication 1, dans lequel l'aiguille hélicoïdale (220) comprend en outre une ou plusieurs fenêtres (322).
